# NEW EUROPEAN PATENT SPECIFICATION

(11) **EP 1 237 615 B2**
(45) Date of publication and mention of the opposition decision: **13.10.2010**
(45) Mention of the grant of the patent: 31.01.2007
(21) Application number: 00987919.8
(22) Date of filing: 18.12.2000
(51) Int. Cl.: A61M 25/00, A61B 19/02

(54) **CATHETER WETTING APPARATUS**
KATHETERBEFEUCHTUNGSVORRICHTUNG
APPAREIL DE MOUILLAGE D'UN CATHETER

(30) Priority: 17.12.1999 SE 9904635
(43) Date of publication of application: 11.09.2002
(73) Proprietor: Astra Tech Aktiebolag, 431 21 Mölndal (SE)
(72) Inventor: NESTENBORG, Daniel, S-430 85 Brännö (SE); UTAS, Jan, S-434 96 Kungsbacka (SE); PETTERSSON, Agneta, S-431 51 Mölndal (SE)
(74) Representative: Lind, Urban Arvid Oskar
(86) International application number: PCT/SE2000/002566
(87) International publication number: WO 2001/043807

(56) References cited:
- WO-A1-97/26937
- WO-A1-98//11932
- GB- - 1 189 855
- GB- - 1 226 364
- US- - 3 255 872

## Description

### Field of the invention

The present invention relates to a wetting apparatus for wetting a hydrophilic urinary catheter, comprising a wetting fluid container containing a wetting fluid and being openable by the application of a pulling force thereto, a wetting receptacle and a hydrophilic urinary catheter to be wetted by said wetting fluid and being arranged within said wetting receptacle. The invention further relates to a method for wetting such a catheter.

### Background of the invention

Intermittent self-catheterisation is widely employed by patients suffering from for example strictures or traumas in the urinary system as well as by paralysed patients to enable the patients to live a nearly normal home life. Urinary catheters supplied for intermittent self-catheterisation in general need to have a lubricant applied to the outer surfaces thereof to facilitate insertion into the urethra. Especially, for lubrication purposes hydrophilic urinary catheters may have a hydrophilic outer surface coating which should be wetted by a fluid such as water or saline for a certain time period prior to insertion thereof into the urethra of a patient.

Various methods for lubricating urinary catheters have been previously proposed, and e.g. the applicant's prior International patent application publication No. W097/26937 discloses a wetting apparatus for wetting hydrophilic urinary catheters. This wetting apparatus comprises a wetting receptacle, which defines a wetting fluid receiving area, a hydrophilic urinary catheter placed in said wetting fluid receiving area, and a wetting fluid container. The wetting fluid container has a discharge outlet which could be opened by application of a pulling force thereto to enable the wetting fluid to be discharged into the wetting fluid receiving area for wetting of the hydrophilic urinary catheter. In this wetting apparatus the receptacle has one open end, in which the fluid container is placed, and with one end protruding out of the receptacle. Further, pulling tabs to apply the pulling force to the container are arranged at each end of the fluid container. To release the fluid, the first pulling tab being inside the receptacle is gripped through the plastic material of the receptacle, and then the second pulling tab being outside the receptacle is pulled rearwardly to cause the container to tear.

Further, a urinary catheter wetting apparatus comprising a receptacle and a rupturable liquid swelling medium pouch or container being placed within the receptacle is known from WO 98/11932. The swelling medium is discharged by applying pressure on the pouch and thereby forcing a rupture on the closure joint.

However, a problem with these known wetting apparatuses is that they are relatively difficult to handle, and especially the discharging of the wetting fluid. Particularly disabled users, such as users with limited capability of moving their limbs, have problems performing the discharging actions needed. Furthermore, it is a problem that the wetting receptacle surrounding the catheter is either opened during the discharge process, or is easily ruptured. This deteriorates the cleanness and sterility of the catheter. There is hereby also a risk for unwanted leakage and spillage of the wetting fluid, which could be very inconvenient for the user.

Accordingly, patients using hydrophilic urinary catheters require improved means for lubricating the catheters. The present invention therefore proposes to address this requirement. This object is achieved with the wetting apparatus and method according to the appended claims.

### Summary of the invention

According to a first aspect of the invention there is provided a wetting apparatus according to claim 1.

Thus, according to the invention the wetting fluid container can be opened and the catheter wetted by the application of a pulling force to the extendable wetting receptacle, without destroying the sealed condition of the wetting receptacle.

By "extendable" is meant that the dimension of the wetting receptacle in a certain pulling direction could be increased without causing damages or destruction to the receptacle to such an extent that the sealed condition of the receptacle is destroyed.

The pulling action for discharging the wetting fluid into the wetting receptacle is a very feasible action, since it is easy to perform and only requires a low pulling force to be exerted. Further, the pulling action could be performed with one hand only, or even without hands, by using the teeth or the like. Thus, this discharging method is suitable for disabled persons, or persons having one or both hands occupied. Thanks to the extendable wetting receptacle this preferred pulling action could be used for discharging the wetting fluid, at the same time as the wetting receptacle could be kept closed and sealed. Hereby, the sterility and cleanness of the catheter could be maintained even during the wetting process, thus reducing the risk of infections for the user. The risk of unwanted leakage and spillage of the wetting liquid is also alleviated.

The wetting fluid container is provided with at least two pulling means for applying a pulling force for opening the container.

By "pulling means" is meant any means providing a transfer of a pulling force applied to the wetting receptacle to the wetting fluid container, such as a portion of the container being fixed to the receptacle.

Further, the wetting fluid container is opened by pulling said pulling means apart in a separation direction, said wetting receptacle being extendable at least in said separation direction. It is also preferred that at least one of the pulling means, and preferably both, is a tab which on application of a predetermined pulling force thereto causes the wetting fluid container to open. These features contribute in making the product simple and inexpensive to manufacture.

The wetting fluid container presents at least one surface area not being fixed to the wetting receptacle and, the wetting fluid container is fixed to the wetting receptacle only in the vicinity of the pulling means. Hereby, as large a part of the wetting receptacle as possible could be used for providing the extendability. Further, the pulling means are preferably fixed to the wetting receptacle by means of welding. However, glue or tape could be used as fixation means as well. Hereby, the manufacture of the product becomes simple and inexpensive.

In one embodiment there is further provided pulling means on the wetting receptacle, such as pulling handles, to facilitate the opening procedure, especially for disabled users.

According to the invention, the wetting receptacle has a surface layer of such dimension as to enable the extendibility of the receptacle. The surface layer of the wetting receptacle has a dimension between the pulling means exceeding the distance between the pulling means, when the wetting fluid container is not opened, and the wetting receptacle comprises a folded section, such as a bellow-like folding.

Preferably, the wetting fluid container presents such a restricted dimension perpendicular to the pulling direction in which said folded section is unfolded, that said unfolding is not restricted by the wetting fluid container. Especially, it is preferred that the wetting receptacle, at said folded section has an inner, cross-sectional dimension perpendicular to the direction of the extension of the receptacle significantly exceeding the corresponding outer, cross-sectional dimension of the wetting fluid container. Thanks to this increased extension, unfoldment of the folded section becomes simple.

The wetting fluid container also comprises an area of weakness, and preferably a tear line, which is opened on the application of the pulling force thereto.

In a preferred embodiment of the invention said area of weakness in the wetting fluid container is arranged in a part of the container facing the catheter in the wetting receptacle. Further, it is most preferred that the wetting fluid container presents a forward edge facing the catheter in the wetting receptacle; that the area of weakness extends rearwardly from the forward edge; and that the wetting container comprises two tabs of which a first tab extends rearwardly from the forward edge on a first side of the area of weakness and is of such dimensions that it rearwardly projects beyond the wetting fluid container, and of which a second tab extends forwardly from the forward edge on a second, opposite side of the area of weakness; wherein the application of a rearward pulling force on the first tab relative to the second tab causes the area of weakness to tear and the wetting fluid container to be opened.

The area of weakness is essentially aligned with the catheter and thus with the elongation of the wetting apparatus, and the pulling direction also coincide with this direction. Hereby, the discharging action becomes simple since essentially the whole extent of the wetting receptacle may be used as a grip.

In preferred embodiments of the invention the fluid container takes the form of a sachet.

It is further preferred that the wetting fluid is sterile water or a saline solution.

A typical sterilising agent which could be used for sterilising the wetting apparatus of the invention is ethylene oxide. Moreover, the fluid in the fluid container would normally be sterile. For these reasons, the wetting fluid container is preferably made of a material which is impermeable or substantially impermeable to ethylene oxide as well as the fluid contained therein. Non-limiting examples of materials satisfying this condition when the fluid is water or saline are aluminium foil laminate, poly(vinylidene chloride) or a laminate comprising metallised film such as metallised poly(ethylene terepthalate), or a silicon oxide coated film.

The wetting receptacle according to the invention may be used only for keeping the catheter clean and sterile and for the described wetting process, and is thereafter disposed. However, in one embodiment the wetting receptacle is also a urine collection bag.

The present invention has the advantage of providing a safe, compact, sterile and disposable wetting apparatus for a hydrophilic urinary catheter, which is easy to handle. This is due to the provision of a wetting receptacle which is adapted to cooperate with a wetting fluid container of the apparatus so as to be able to release the fluid into the wetting receptacle to wet a hydrophilic urinary catheter placed therein under clean conditions, that is to say, without the need for touching the catheter or the fluid, or even breaking the receptacle. Hereby, the risk of introducing contaminants is avoided, and a wetting apparatus that is easy to handle as well as to manufacture is provided.

According to the invention, the receptacle has an inherent elongation, enabling bringing of the receptacle from the first to the second position, and preferably this inherent elongation comprises a bellow-like folding. Hereby, the expandability is achieved without any special requirements on the material, which makes the manufacture simple and inexpensive.

It is further preferred that the receptacle, in the area of said folding, has an inner, cross-sectional extension perpendicular to the direction of said inherent elongation significantly exceeding the corresponding outer, cross-sectional extension of the wetting fluid container.

According to a third aspect of the invention, there is provided a method according to claim 14.

In an embodiment according to this third aspect of the invention said transfer of the pulling force is accomplished by an elongation of the wetting receptacle. The transfer of the pulling force is accomplished by the use of at least two pulling means being fixed to the wetting receptacle, whereby the pulling force is applied in such a way that the pulling means are pulled apart.

### Brief description of the drawings

By way of example embodiments of the invention will now be described with reference to the accompanying drawings in which:
Fig. 1 shows a wetting apparatus according to an embodiment of the invention comprising a wetting receptacle and an unopened wetting fluid sachet.
Fig. 2 shows the part of the wetting apparatus shown in Fig. 1 comprising the unopened sachet more in detail.
Fig. 3 shows the wetting apparatus shown in fig 1 where the wetting fluid sachet is in the opened position.
Fig. 4 shows the part of the wetting apparatus shown in Fig. 3 comprising the unopened sachet more in detail.
Fig. 5 shows a wetting apparatus according to another embodiment of the invention.

### Description of preferred embodiments

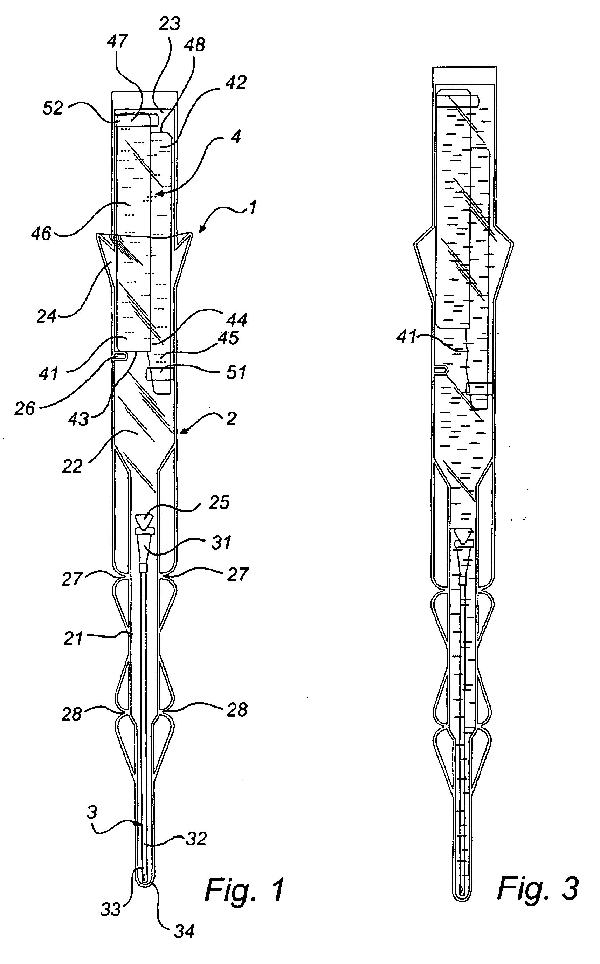

Referring first to Fig. 1, there is shown a first embodiment of a wetting apparatus 1 according to the invention comprising a wetting receptacle, or bag 2, preferably of a transparent flexible plastics material. The receptacle 2 has a downwardly extending elongate pocket 21 at the forward end, an intermediate chamber 22 rearwardly of and in fluid communication with the elongate pocket 21 and a fluid supply chamber 23 spaced further rearwardly.

The wetting apparatus further comprises a hydrophilic urinary catheter 3 having a flared rearward portion 31, an elongate shaft 32 projecting forwardly from the rearward portion 31 and an open-ended lumen (not shown) which extends from the rear end of the rearward portion 31 to a drainage aperture 33 in the rounded tip 34. The rearward end 31 of the catheter 3 is connectable to a urine collection bag.

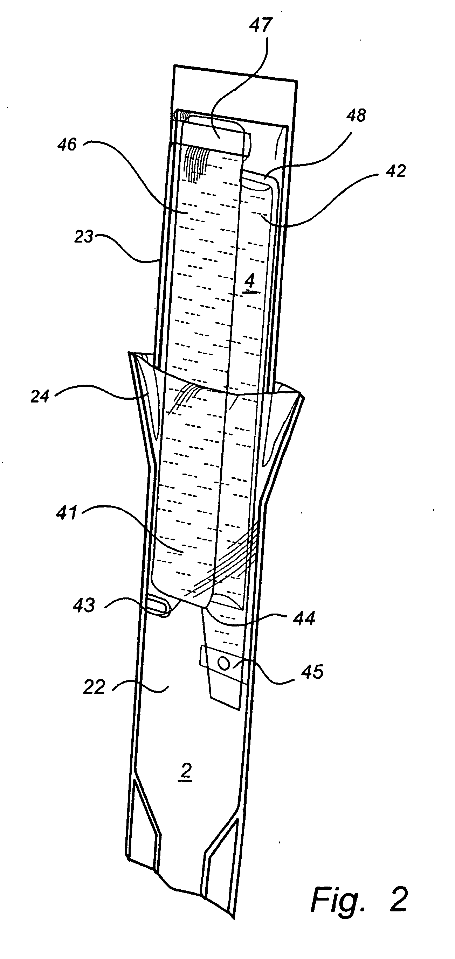

Further, the wetting apparatus comprises a wetting fluid container 4, which in this embodiment takes the form of a sachet, containing a wetting fluid. As can be seen more particularly by reference to Fig. 2, the sachet 4 is arranged in the fluid supply chamber 23 in an operational position. The sachet 4 has a forward portion 41 which in the operational position of the sachet 4 faces the catheter and the fluid receiving area 21 and a rearward portion 42 which in the operational position projects rearwardly away from the catheter and the fluid receiving area 21. The sachet 4 is preferably made of aluminium foil laminate, poly(vinylidene chloride) or a laminate containing a metallised film, such as metallised poly(ethylene terepthalate), or a silicon oxide coated film, particularly when ethylene oxide is the sterilising agent for the apparatus 1 and the sachet contains sterile water or saline solution.

Preferably, the forward portion 41 of the sachet 4 presents a forward edge 43. Extending rearwardly from the forward edge 43 is an area of weakness, and preferably a tear line 44. Projecting forwardly from the forward edge 43 of the sachet to one side of the tear line 44 is a first tab 45. On the other side of the tear line 44 there is provided an elongate second tab 46, shown here in an extended position in which the second tab 46 projects forwardly from the forward edge 43. The elongate second tab 46 is movable about the forward edge 43 back on it self from the extended position to a retracted position in which the second tab 46 extends rearwardly from the forward edge 43. When the second tab 46 is in the retracted position the sachet 4 is inserted into the fluid supply chamber into the operational position shown in Fig 1.

Preferably the dimensions of the second tab 46 are such that when the sachet 4 is in the operational position a pulling portion 47 of the second tab 46 projects rearwardly beyond a rearward edge 48 of the sachet 4.

The sachet is fixed to the receptacle, but most preferably only in the vicinity of the pulling means, i.e. the tabs 45,46. The fixation could be provided by means of welding, gluing or taping, or a combination of these, but other fixation means could be used as well. Between the fixation points the fluid supply chamber 23 of the receptacle is provided with a surplus of material, resulting in bellow-like folded area 24. This folded area makes an easy elongation of the fluid supply chamber possible. The folded area is further preferably wider than the rest of the fluid supply chamber 23.

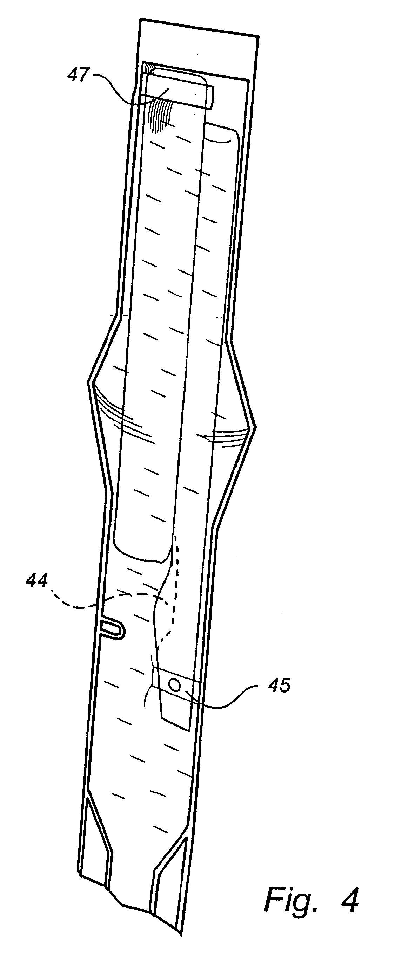

In figs 3 and 4 the sachet 4 is shown in its opened position, where the contents of the sachet is released into the receptacle 2 to wet the hydrophilic outer coating of the catheter 3.

In the method of wetting the catheter, the user applies a pulling force to the wetting receptacle in such a way that the pulling force is transferred to the wetting container to open the same without rupturing the sealed condition of the wetting receptacle. The pulling force is applied in such a way that the pulling means are pulled apart in a separation direction. This could e.g. be made in the following way, with the wetting apparatus described above. The user grips the first tab 45 through the flexible transparent plastics material of the bag 2 and then pulls rearwardly on the pulling portion 47 of the second tab 46, likewise through the flexible transparent plastics material of the bag 2, to cause the tear line 44 to be torn and the wetting fluid to be released into the pocket 21 to wet the catheter 3. Preferably, the sachet 4 contains a sufficient amount of wetting fluid for the pocket 21 to be filled to a level, which results in the insertable length of the catheter 3 being wetted. By insertable length" is meant at least that length of the elongate shaft 32 which is coated with a hydrophilic material, for example PVP, and inserted into the urethra of the patient. Typically, this will be 80-140mm for a female patient and 200-350mm for a male patient.

After release of the wetting fluid into the pocket 21 the receptacle is opened, preferably at a tearing section 27, where after the catheter is removed from the bag 21 and used for catherisation. The receptacle and sachet are then disposed of. To facilitate the removal of the catheter from the receptacle and the insertion into the urethra of the patient, at least one area of weakness 27, 28, such as a tear line, is preferably arranged on the receptacle in the area of fluid receiving pocket 21, in which the catheter is placed. Most preferably, two such areas of weakness 27, 28 are provided, and separated in the lengthwise direction of the receptacle. The intermediate part of the receptacle may be used as an insertion aid for guiding and holding the wetted catheter when it is inserted into the urethra. There is therefore no need to directly handle the catheter 3 for insertion thereof into the urethra, which is to advantage as the outer surface of the catheter 3 will be slippery due to the wetting procedure and therefore difficult to grip and furthermore because the possibility of contamination of the catheter 3 at this stage is avoided, whereby the cleanness and sterility of the catheter may be maintained.

The bag or receptacle 2 according to the invention is a closed bag with the sachet 4 and catheter 3 prepackaged within the bag 2.

The catheter 3 could be sterilised using ethylene oxide. Since the sachet 4 contains sterile water or saline there is no need for sterilising the contents of the sachet 4. Accordingly, the material of the sachet 6 is preferably impermeable to ethylene oxide and water. Non-limiting examples of materials meeting these requirements are poly(vinylidene chloride) (PVDC), aluminium foil laminates or a laminate comprising a metallised film, for example metallised poly(ethylene terepthalate), or a silicon coated film. Other sterilisation processes could of course be used instead, for example by irradiation in which case the fluid in the sachet 4 could be sterilised in situ at the same time as the rest of the components of the apparatus 1. Steam treatment may also be used for sterilisation.

Other types and locations of sachets 4 inside the bag is possible as long as the sachet 4 releases its contents into the pocket 21.

Even though the receptacle according to the invention is sealed, it is however preferred that the receptacle 2 is provided with an outlet 25, at least when ethylene oxide is the sterilising agent, as this provides a pathway for the ethylene oxide to enter and exit the inside of the receptacle.

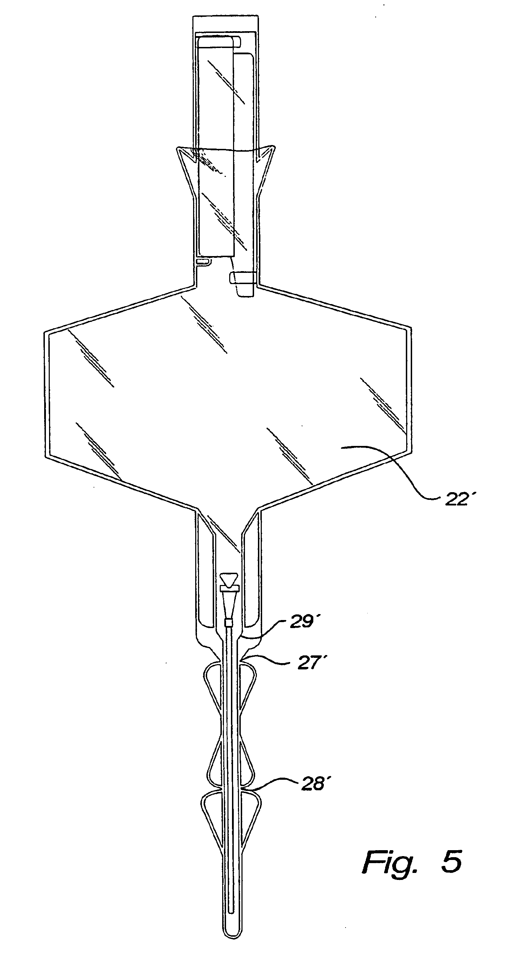

In still another embodiment, shown in fig 5, the intermittent chamber 22' of the receptacle 2 may be used as a urine bag. In this case, after wetting of the catheter 3 for the predetermined duration in the same manner as described above, the bag 2 is turned upside down and the forwardmost portion of the pocket 21 torn off. The elongate shaft 32 of the catheter 3 is then maneuvered through the opening in the forward end of the pocket 21 and pulled out until the flared rearward portion 31 forms a mechanical seal connection with the opening at a restriction 29' of the receptacle. Thereafter the catheter is inserted into the urethra of the patient. Two separated areas of weakness 27', 28' may be used as an insertion aid even in this embodiment. However, in this case the areas of weakness should be placed beneath the restriction 29'. Except the above mentioned differences, the same as discussed above regarding the first embodiments applies to this alternative embodiment as well.

In the exemplary embodiments hereinabove described with reference to drawings the supply of wetting fluid for wetting of the hydrophilic urinary catheter takes the form of a separate sachet integrated into the wetting receptacle, and being easily dischargeable without breaking or rupturing the receptacle. It will be appreciated by those versed in the art that several alternatives similar to those described above could be used, such as other types of fluid containers, different ways to provide the expandability of the receptacle etc.

## Claims

1. A wetting apparatus (1) for wetting a hydrophilic urinary catheter (3), comprising:
a wetting fluid container (4) containing a wetting fluid and being openable by the application of a pulling force thereto;
a wetting receptacle (2); and
a hydrophilic urinary catheter (3) to be wetted by said wetting fluid and being arranged within said wetting receptacle (2), **characterised in that** the wetting fluid container (4) is arranged within the wetting receptacle (2), and that the wetting receptacle is extendable, for opening the wetting container without rupturing a sealed condition of the wetting receptacle, wherein the wetting fluid container is provided with at least two pulling means (45,46) for applying said pulling force for opening the container, the wetting fluid container (4) being fixed to the wetting receptacle (2) only in the vicinity of the pulling means (45,46) and having at least one surface area not being fixed to the wetting receptacle (2), and wherein the wetting receptacle (2) has a surface layer of such dimension between the pulling means (45,46) that it exceeds the distance between the pulling means when the wetting fluid container is not opened, whereby the wetting receptacle (2) comprises at least one folded section (24), such as a bellow-like folding, thereby enabling the extendibility of the receptacle, the wetting fluid container being openable by pulling said pulling means (45,46) apart in a separation direction and said wetting receptacle (2) being extendable at least in said separation direction, and wherein the wetting fluid container (4) comprises an area of weakness (44), preferably a tear line, which is opened on the application of the pulling force thereto, said area of weakness extending in a direction coinciding with said separation direction, and also with the direction of the catheter.

2. A wetting apparatus according to claim 1, wherein at least one of the pulling means, and preferably both, is a tab (45,46) which on application of a predetermined pulling force thereto causes the wetting fluid container (4) to open.

3. A wetting apparatus according to any one of the preceding claims, wherein the wetting fluid container (4) is fixed to the wetting receptacle (2) by means of welding.

4. A wetting apparatus according to any one of the preceding claims, wherein the wetting receptacle (2) comprises pulling means, such as pulling handles, to facilitate opening of the wetting fluid container (4).

5. A wetting apparatus according to any one of the preceding claims, wherein the wetting receptacle (2) has such a restricted dimension perpendicula to the pulling direction in which said folded section (24) is unfolded, that said unfolding is not restricted by the wetting fluid container (4).

6. A wetting apparatus according to claim 5, wherein the wetting receptacle (2), at said folded section (24) has an inner, cross-sectional dimension perpendicular to the extension direction of the receptacle (4) significantly exceeding the corresponding outer, cross-sectional dimension of the wetting fluid container.

7. A wetting apparatus according to claim 1, wherein the area of weakness (44) in the wetting fluid container (4) is arranged in a part of the container facing the catheter (3) in the wetting receptacle (2).

8. A wetting apparatus according to claim 7, wherein the wetting fluid container (4) presents a forward edge (43) facing the catheter (3) in the wetting receptacle (2); the area of weakness (44) extends rearwardly from the forward edge (43); the wetting container comprises two tabs, forming pulling means for applying said pulling force for opening the container on application of a predetermined pulling force thereto, of which a first tab (46) extends rearwardly from the forward edge on a first side of the area of weakness and is of such dimensions that it rearwardly projects beyond the wetting fluid container, and of which a second tab (45) extends forwardly from the forward edge on a second, opposite side of the area of weakness (44); wherein the application of a rearward pulling force on the first tab (46) relative to the second tab (45) causes the area of weakness (44) to tear and the wetting fluid container to be opened.

9. A wetting apparatus according to any one of the preceding claims, wherein the wetting fluid container (4) takes the form of a sachet.

10. A wetting apparatus according to any one of the preceding claims, wherein the wetting fluid is sterile water or a saline solution.

11. A wetting apparatus according to claim 10, wherein the wetting fluid container (4) is impermeable to ethylene oxide and water or saline solution.

12. A wetting apparatus according to any one of the preceding claims, wherein the wetting fluid container (4) is made of aluminium foil laminate, poly(vinylidene chloride) or a laminate comprising a metallised film, such as metallised poly(ethylene terepthalate), or a silicon oxide coated film.

13. A wetting apparatus according to any one of the claims above, wherein the wetting receptacle (2) is a urine collection bag.

14. A method for wetting a hydrophilic urinary catheter (3) placed in a wetting receptacle (2) together with a wetting fluid container (4) containing a wetting fluid, **characterised by** the step of applying a pulling force to the wetting receptacle (2), to extend the receptacle without opening the latter, for transferring the pulling force via the wetting receptacle to the wetting container (4) to open the latter and discharging the wetting fluid from the thus opened wetting fluid container into the still unopened wetting receptacle, wherein said transfer of the pulling force is accomplished by the use of at least two pulling means (45,46) being fixed to the wetting receptacle (2), the wetting fluid container (4) being fixed to the wetting receptacle (2) only in the vicinity of the pulling means (45,46) and having at least one surface area not being fixed to the wetting receptacle (2), whereby the pulling force is applied in such a way that the pulling means are pulled apart, and wherein the wetting receptacle (2) has a surface layer of such dimension between the pulling means (45,46) that it exceeds the distance between the pulling means when the wetting fluid container is not opened, whereby the wetting receptacle (2) comprises at least one folded section (24), such as a bellow-like folding, thereby enabling the extendibility of the receptacle, the wetting fluid container being openable by pulling said pulling means (45,46) apart in a separation direction and said wetting receptacle (2) being extendable at least in said separation direction, and wherein the wetting fluid container (4) comprises an area of weakness (44), preferably a tear line, which is opened on the application of the pulling force thereto, said area of weakness extending in a direction coinciding with said separation direction, and also with the direction of the catheter.

## Patentansprüche

1. Befeuchtungsvorrichtung (1) zum Befeuchten eines hydrophilen Harnkatheters (3) mit:
einem Befeuchtungsfluidbehälter (4), der ein Befeuchtungsfluid enthält und geöffnet werden kann, indem eine Zugkraft auf ihn ausgeübt wird;
einem Befeuchtungsbehältnis (2); und
einem hydrophilen Harnkatheter (3), der durch das Befeuchtungsfluid befeuchtet werden soll und im Befeuchtungsbehältnis (2) angeordnet ist, **dadurch gekennzeichnet, dass** der Befeuchtungsfluidbehälter (4) im Befeuchtungsbehältnis (2) angeordnet ist und das Befeuchtungsbehältnis dehnbar ist, um den Befeuchtungsbehälter ohne ein Verletzen des dicht verschlossenen Zustandes des Befeuchtungsbehältnisses zu öffnen, wobei der Befeuchtungsfluidbehälter mit zumindest zwei Zugelementen (45, 46) versehen ist, um die Zugkraft zum Öffnen des Behälters anzulegen, der Befeuchtungsfluidbehälter (4) nur in der Nähe der Zugelemente (45, 46) am Befeuchtungsbehältnis (2) befestigt ist und zumindest einen nicht am Befeuchtungsbehältnis (2) befestigten Oberflächenbereich hat, und wobei das Befeuchtungsbehältnis (2) eine Oberflächenschicht zwischen den Zugelementen (45, 46) mit einer solchen Abmessung hat, dass sie den Abstand zwischen den Zugelementen übertrifft, wenn der Befeuchtungsfluidbehälter nicht geöffnet ist, wobei das Befeuchtungsbehältnis (2) zumindest einen gefalteten Abschnitt (24) wie z.B. eine balgartige Faltung umfasst, um das Behältnis dehnbar zu machen, wobei der Befeuchtungsfluidbehälter geöffnet werden kann, indem die Zugelemente (45, 46) in einer trennenden Richtung auseinander gezogen werden, wobei das Befeuchtungsbehältnis (2) zumindest in der trennenden Richtung dehnbar ist, und wobei der Befeuchtungsfluidbehälter (4) eine Fläche mit einer Schwachstelle (44) umfasst, bevorzugt eine Reißlinie, die geöffnet wird, wenn die Zugkraft daran angelegt wird, wobei sich die Fläche mit einer Schwachstelle in eine Richtung erstreckt, die mit der trennenden Richtung und auch mit der Richtung des Katheters übereinstimmt.

2. Befeuchtungsvorrichtung nach Anspruch 1, wobei zumindest eines der Zugelemente, bevorzugt beide, eine Lasche (45, 46) ist, die ein Öffnen des Befeuchtungsfluidbehälters (4) bewirkt, wenn eine vorbestimmte Zugkraft daran angelegt wird.

3. Befeuchtungsvorrichtung nach einem der vorangehenden Ansprüche, wobei der Befeuchtungsfluidbehälter (4) durch Schweißen am Befeuchtungsbehältnis (2) befestigt ist.

4. Befeuchtungsvorrichtung nach einem der vorangehenden Ansprüche, wobei das Befeuchtungsbehältnis (2) Zugelemente wie Zuggriffe umfasst, um das Öffnen des Befeuchtungsfluidbehälters (4) zu erleichtern.

5. Befeuchtungsvorrichtung nach einem der vorangehenden Ansprüche, wobei das Befeuchtungsbehältnis (2) senkrecht zu der Zugrichtung, in der der gefaltete Abschnitt (24) entfaltet wird, eine derart beschränkte Abmessung hat, dass das Entfalten durch den Befeuchtungsfluidbehälter (4) nicht beschränkt wird.

6. Befeuchtungsvorrichtung nach Anspruch 5, wobei das Befeuchtungsbehältnis (2) am gefalteten Abschnitt (24) eine innere Querschnittsabmessung senkrecht zur Dehnungsrichtung des Behältnisses (4) hat, die die entsprechende äußere Querschnittsabmessung des Befeuchtungsfluidbehälters signifikant übertrifft.

7. Befeuchtungsvorrichtung nach Anspruch 1, wobei die Fläche mit Schwachstelle (44) im Befeuchtungsfluidbehälter (4) in einem Teil des Behälters angeordnet ist, der dem Katheter (3) im Befeuchtungsbehältnis (2) zugewandt ist.

8. Befeuchtungsvorrichtung nach Anspruch 7, wobei der Befeuchtungsfluidbehälter (4) eine Vorderkante (43) aufweist, die dem Katheter (3) im Befeuchtungsbehältnis (2) zugewandt ist; die Fläche mit Schwachstelle (44) sich von der Vorderkante (43) nach hinten erstreckt; der Befeuchtungsbehälter zwei Laschen umfasst, die Zugelemente bilden, um die Zugkraft anzulegen, um den Behälter zu öffnen, wenn eine vorbestimmte Zugkraft daran angelegt wird, wovon sich eine erste Lasche (46) von der Vorderkante auf einer ersten Seite der Fläche mit Schwachstelle nach hinten erstreckt und so bemessen ist, dass sie rückwärts über den Befeuchtungsfluidbehälter hinausragt, und wovon sich eine zweite Lasche (45) von der Vorderkante auf einer zweiten, entgegen gesetzten Seite der Fläche mit Schwachstelle (44) nach vorn erstreckt; wobei die Anwendung einer bezüglich der zweiten Lasche (45) nach hinten ziehenden Kraft an die erste Lasche (46) bewirkt, dass die Fläche mit Schwachstelle (44) reißt und der Befeuchtungsfluidbehälter sich öffnet.

9. Befeuchtungsvorrichtung nach einem der vorangehenden Ansprüche, wobei der Befeuchtungsfluidbehälter (4) die Gestalt eines Beutels annimmt.

10. Befeuchtungsvorrichtung nach einem der vorangehenden Ansprüche, wobei das Befeuchtungsfluid steriles Wasser oder eine Salzlösung ist.

11. Befeuchtungsvorrichtung nach Anspruch 10, wobei der Befeuchtungsfluidbehälter (4) gegenüber Ethylenoxid und Wasser bzw. Salzlösung undurchlässig ist.

12. Befeuchtungsvorrichtung nach einem der vorangehenden Ansprüche, **dadurch gekennzeichnet, dass** der Befeuchtungsfluidbehälter (4) aus einem Aluminiumfolienlaminat, aus Polyvinylidenchlorid oder einem Laminat mit einer Metallisierungsschicht wie metallisiertem Polyethylenterephthalat oder aus einer mit Siliciumoxid beschichteten Fol ie besteht.

13. Befeuchtungsvorrichtung nach einem der vorangehenden Ansprüche, wobei das Befeuchtungsbehältnis (2) ein Harnsammelbeutel ist.

14. Verfahren zur Befeuchtung eines hydrophilen Harnkatheters (3), der zusammen mit einem das Befeuchtungsfluid enthaltenden Befeuchtungsfluidbehälter (4) in einem Befeuchtungsbehältnis (2) untergebracht ist, **gekennzeichnet durch** den Schritt, eine Zugkraft auf das Befeuchtungsbehältnis (2) auszuüben, um das Behältnis auszudehnen ohne es zu öffnen, um die Zugkraft über das Befeuchtungsbehältnis auf den Befeuchtungsbehälter (4) zu übertragen, um letzteren zu öffnen und das Befeuchtungsfluid aus dem so geöffneten Befeuchtungsfluidbehälter in das noch ungeöffnete Befeuchtungsbehältnis zu entleeren, wobei die Übertragung der Zugkraft **durch** die Verwendung von mindestens zwei Zugelementen (45, 46) erreicht wird, die am Befeuchtungsbehältnis (2) befestigt sind, der Befeuchtungsfluidbehälter (4) nur in der Nähe der Zugelemente (45, 46) am Befeuchtungsbehältnis (2) befestigt ist und zumindest einen nicht am Befeuchtungsbehältnis (2) befestigten Oberflächenbereich hat, wodurch die Zugkraft derart angelegt wird, dass die Zugelemente auseinander gezogen werden, und wobei das Befeuchtungsbehältnis (2) eine Oberflächenschicht zwischen den Zugelementen (45, 46) mit einer solchen Abmessung hat, dass sie den Abstand zwischen den Zugelementen übertrifft, wenn der Befeuchtungsfluidbehälter nicht geöffnet ist, wobei das Befeuchtungsbehältnis (2) zumindest einen gefalteten Abschnitt (24) wie z.B. eine balgartige Faltung umfasst, um das Behältnis dehnbar zu machen, wobei der Befeuchtungsfluidbehälter geöffnet werden kann, indem die Zugelemente (45, 46) in einer trennenden Richtung auseinander gezogen werden, wobei das Befeuchtungsbehältnis (2) zumindest in der trennenden Richtung dehnbar ist, und wobei der Befeuchtungsfluidbehälter (4) eine Fläche mit einer Schwachstelle (44) umfasst, bevorzugt eine Reißlinie, die geöffnet wird, wenn die Zugkraft daran angelegt wird, wobei sich die Fläche mit einer Schwachstelle in eine Richtung erstreckt, die mit der trennenden Richtung und auch mit der Richtung des Katheters übereinstimmt.

## Revendications

1. Appareil (1) de mouillage destiné à mouiller un cathéter (3) urinaire hydrophile, comprenant :
un conteneur (4) de fluide de mouillage contenant un fluide de mouillage et pouvant s'ouvrir par l'application d'une force de tirage sur celui-ci ;
un réceptacle (2) de mouillage ; et
un cathéter (3) urinaire hydrophile à humidifier avec ledit fluide de mouillage et étant disposé dans ledit réceptacle (2) de mouillage, **caractérisé en ce que** le conteneur (4) de fluide de mouillage est disposé dans le réceptacle (2) de mouillage, et **en ce que** le réceptacle de mouillage est extensible, pour ouvrir le conteneur de mouillage sans rompre un état hermétique du réceptacle de mouillage, dans lequel le conteneur de fluide de mouillage est muni d'au moins deux moyens (45, 46) de tirage destinés à appliquer ladite force de tirage pour ouvrir le conteneur, le conteneur (4) de fluide de mouillage étant fixé au réceptacle (2) de mouillage seulement en voisinage des moyens (45, 46) de tirage et ayant au moins une zone de surface qui n'est pas fixée au réceptacle (2) de mouillage, et dans lequel le réceptacle (2) de mouillage présente une couche de surface d'une dimension entre les moyens (45, 46) de tirage dépassant la distance entre les moyens de tirage, lorsque le conteneur de fluide de mouillage n'est pas ouvert, dans lequel le réceptacle (2) de mouillage comprend au moins une section (24) pliée, telle qu'une pliure de type soufflet, afin de rendre le réceptacle extensible, le conteneur de fluide de mouillage pouvant s'ouvrir en séparant lesdits moyens (45, 46) de tirage dans une direction de séparation et ledit réceptacle (2) de mouillage étant extensible au moins dans ladite direction de séparation et dans lequel le conteneur (4) de fluide de mouillage comprend une zone d'affaiblissement (44), de préférence une ligne de déchirure, qui est ouverte par l'application de la force de tirage sur celle-ci, ladite zone d'affaiblissement s'étendant dans une direction coïncidant avec ladite direction de séparation et également avec la direction du cathéter.

2. Appareil de mouillage selon la revendication 1, dans lequel au moins un des moyens de tirage, et de préférence les deux, est une languette (45, 46) qui entraîne l'ouverture du conteneur (4) de fluide de mouillage par l'application d'une force de tirage prédéterminée sur celle-ci.

3. Appareil de mouillage selon l'une quelconque des revendications précédentes, dans lequel le conteneur (4) de fluide de mouillage est fixé au réceptacle (2) de mouillage par soudure.

4. Appareil de mouillage selon l'une quelconque des revendications précédentes, dans lequel le réceptacle (2) de mouillage comprend des moyens de tirage, tels que des poignées de tirage, afin de faciliter l'ouverture du conteneur (4) de fluide de mouillage.

5. Appareil de mouillage selon l'une quelconque des revendications précédentes, dans lequel le réceptacle (2) de mouillage présente une telle dimension limitée perpendiculaire à la direction de tirage dans laquelle ladite section (24) pliée est dépliée, que ledit dépliage n'est pas limité par le conteneur (4) de fluide de mouillage.

6. Appareil de mouillage selon la revendication 5 dans lequel le réceptacle (2) de mouillage, à ladite section (24) pliée, présente une dimension de la section transversale intérieure perpendiculaire à la direction d'extension du réceptacle (4) dépassant de manière significative la dimension de la section transversale extérieure correspondante du conteneur de fluide de mouillage.

7. Appareil de mouillage selon la revendication 1, dans lequel la zone d'affaiblissement (44) dans le conteneur (4) de fluide de mouillage est disposée dans une partie du conteneur faisant face au cathéter (3) dans le réceptacle (2) de mouillage.

8. Appareil de mouillage selon la revendication 7, dans lequel le conteneur (4) de fluide de mouillage présente un bord orienté vers l'avant (43) faisant face au cathéter (3) dans le réceptacle (2) de mouillage; la zone d'affaiblissement (44) s'étend vers l'arrière depuis le bord orienté vers l'avant (43) ; le conteneur de mouillage comprend deux languettes, formant des moyens de tirage destinés à appliquer ladite force de tirage pour ouvrir le conteneur par l'application d'une force de tirage prédéterminée sur celles-ci, dont une première languette (46) s'étend vers l'arrière depuis le bord orienté vers l'avant sur un premier côté de la zone d'affaiblissement et a des dimensions telles qu'elle fait saillie vers l'arrière au-delà du conteneur de fluide de mouillage, et dont une deuxième languette (45) s'étend vers l'avant depuis le bord orienté vers l'avant sur un deuxième côté opposé de la zone d'affaiblissement (44) ; dans lequel l'application d'une force de tirage vers l'arrière sur la première languette (46) par rapport à la deuxième languette (45) entraîne la déchirure de la zone d'affaiblissement (44) et l'ouverture du conteneur de fluide de mouillage.

9. Appareil de mouillage selon l'une quelconque des revendications précédentes, dans lequel le conteneur (4) de fluide de mouillage a la forme d'un sachet.

10. Appareil de mouillage selon l'une quelconque des revendications précédentes, dans lequel le fluide de mouillage est de l'eau stérile ou une solution saline.

11. Appareil de mouillage selon la revendication 10, dans lequel le conteneur (4) de fluide de mouillage est imperméable à l'oxyde d'éthylène ainsi qu'à une solution aqueuse ou saline.

12. Appareil de mouillage selon l'une quelconque des revendications précédentes, dans lequel le conteneur (4) de fluide de mouillage est constitué d'un laminé de feuille aluminium, du poly(chlorure de vinylidène) ou d'un laminé comprenant un film métallisé, tel que du poly(éthylène téréphtalate) métallisé, ou d'un film revêtu d'oxyde de silicium.

13. Appareil de mouillage selon l'une quelconque des revendications ci-dessus, dans lequel le réceptacle (2) de mouillage est un sac de récupération d'urine.

14. Procédé destiné à humidifier un cathéter (3) urinaire hydrophile placé dans un réceptacle (2) de mouillage avec un conteneur (4) de fluide de mouillage contenant un fluide de mouillage, **caractérisé par** l'étape consistant à appliquer une force de tirage au réceptacle (2) de mouillage, pour étirer le réceptacle sans ouvrir ce dernier, pour transférer la force de tirage par l'intermédiaire du réceptacle de mouillage au conteneur (4) de mouillage afin d'ouvrir ce dernier et pour évacuer le fluide de mouillage depuis le conteneur de fluide de mouillage ainsi ouvert dans le réceptacle de mouillage toujours non ouvert dans lequel ledit transfert de la force de tirage est obtenu par l'utilisation d'au moins deux moyens (45, 46) de tirage fixés au réceptacle (2) de mouillage, le conteneur (4) de fluide de mouillage étant fixé au réceptacle (2) de mouillage seulement en voisinage des moyens (45, 46) de tirage et ayant au moins une zone de surface qui n'est pas fixée au réceptacle (2) de mouillage, d'où il résulte que la force de tirage est appliquée de telle sorte que les moyens de tirage sont séparés et dans lequel le réceptacle (2) de mouillage présente une couche de surface d'une dimension entre les moyens (45, 46) de tirage dépassant la distance entre les moyens de tirage, lorsque le conteneur de fluide de mouillage n'est pas ouvert, dans lequel le réceptacle (2) de mouillage comprend au moins une section (24) pliée, telle qu'une pliure de type soufflet, afin de rendre le réceptacle extensible, le conteneur de fluide de mouillage pouvant s'ouvrir en séparant lesdits moyens (45, 46) de tirage dans une direction de séparation et ledit réceptacle (2) de mouillage étant extensible au moins dans ladite direction de séparation et dans lequel le conteneur (4) de fluide de mouillage comprend une zone d'affaiblissement (44), de préférence une ligne de déchirure, qui est ouverte par l'application de la force de tirage sur celle-ci, ladite zone d'affaiblissement s'étendant dans une direction coïncidant avec ladite direction de séparation et également avec la direction du cathéter.
